(19) Europäisches Patentamt
European Patent Office
Office européen des brevets

(11) **EP 1 921 436 A2**

(12) **EUROPÄISCHE PATENTANMELDUNG**

(43) Veröffentlichungstag:
**14.05.2008 Patentblatt 2008/20**

(51) Int Cl.:
***G01N 11/16*** *(2006.01)*

(21) Anmeldenummer: 07119997.0

(22) Anmeldetag: **05.11.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **10.11.2006 DE 102006053390**

(71) Anmelder: **LANXESS Deutschland GmbH 51369 Leverkusen (DE)**

(72) Erfinder:
- **Wrana, Claus
 51061, Köln (DE)**
- **Kroll, Jochen
 52066, Aachen (DE)**

(54) **Verfahren zur Bestimmung einer Molekulargewichtsverteilung in einem Polymer**

(57) Die Erfindung betrifft ein Verfahren zur Bestimmung einer Molekulargewichtsverteilung in einem Polymer. Wird ein Polymer hergestellt, so entstehen Ketten unterschiedlicher Länge. Entsprechend ergibt sich eine Verteilung der Molekulargewichte der Ketten eines Polymers.

Es ist Aufgabe der Erfindung, die Molekulargewichtsverteilung im Vergleich zum Stand der Technik verbessert ermitteln zu können.

Die Aufgabe der Erfindung wird gelöst, indem numerisch zur Verfügung stehende Ausgangsdaten $G^*(\omega)$, also das Frequenz abhängig gemessene komplexes Schermodul oder die hieraus konstruierte Masterkurve, durch eine analytische Funktion beschrieben werden. Auf diese Weise steht in verbesserter Form eine genaue Kenntnis des Relaxatiansverilaltens zur Verfügung und zwar vor allem auch bei längeren Reiaxationszeiten. Dies ermöglicht eine zutreffende Ermittlung des Relaxationszeitspektrums und damit eine grundsätzlich zutreffendere Berechnung der Molekulargewichtsverteilung.

EP 1 921 436 A2

**Beschreibung**

Die Erfindung betrifft ein Verfahren zur Bestimmung einer Molekulargewichtsverteilung in einem Polymer, Wird ein Polymer hergestellt, so entstehen Ketten unterschiedlicher Länge. Entsprechend ergibt sich eine Verteilung der Molekulargewichte der Ketten eines Polymers.

Aus dem Stand der Technik sind verschiedene Verfahren bekannt, um eine Molekulargewichtsverteilung zu bestimmen, so zum Beispiel unter der Bezeichnung "GPC". Es werden bei dem GPC-Verfahren ein Polymer gelöst und auf eine poröse Säule aufgegeben, Je schneller die Säule passiert wird, umso höher ist das Molekulargewicht. Die Geschwindigkeit, mit der die Säule durchlaufen wird, ist also ein Maß für die Molekulargewichtsverteilung.

Dieses Verfahren weist den Nachteil auf, nur solche Polymere erfassen zu können, die löslich sind. Auch nimmt die Empfindlichkeit des Verfahrens mit größer werdendem Molekulargewicht ab.

Um den Nachteil bezüglich der Empfindlichkeit mit zunehmendem Molekulargewicht zu überwinden, wird nach dem Stand der Technik vorgeschlagen, eine Molekulargewichtsverteilung auf der Grundlage von physikalischen Daten zu berechnen. Ein solches Verfahren ist u. a. aus der Druckschrift "W. Thimm et al., An analytical relation between relaxation time spectrum and molecular weight distribution, J. Rheol, 43 No. 6 (1999) 1663-1672" bekannt geworden.

Zunächst wird das komplexe Schermodul bei verschiedenen Temperaturen frequenzabhängig gemessen. Aus technischen Gründen wird bei Frequenzen von $10^{-3}$ bis $10^3$ Hz gemessen, Eine Unterschreitung des Wertes von $10^{-3}$ Hz lässt die Messdauer stark anwachsen. So beträgt die Messdauer bei $10^{-3}$ Hz beispielsweise 1½ Stunden. Aus apparativen Gründen sind Messungen oberhalb von $10^3$ Hz problematisch, da Eigenschwingungen des apparativen Aufbaus das Ergebnis verfälschen.

Um unabhängig von diesem Problem Daten über einen größeren Frequenzbereich auswerten zu können, wird ausgenutzt, dass eine Messung bei tieferen Temperaturen in eine Messung bei höheren Frequenzen umgerechnet werden kann. Es resultiert aus einer solchen Umrechnung eine sogenannte Masterkurve, die ein breiteres Frequenzspektrum abdeckt. Es liegen dann in numerischer Form zwei Funktionen vor, die das komplexe Schermodul gegenüber der Frequenz charakterisieren bzw. wiedergeben. Es handelt sich zum einen um den Realteil und zum anderen um den Imaginärteil. Der Realteil wird Speichermodul und der Imaginärteil wird Verlustmodul genannt, Der typische Frequenzbereich umschließt nun 14 - 20 Dekaden, selbst wenn nur innerhalb des vorgenannten Frequenzbereichs gemessen wurde.

Nach dem Stand der Technik wird numerisch hieraus das sogenannte Relaxationszeitspektrum ermittelt. Die numerische Umrechnung von numerischen Ausgangsdaten in ein Relaxationszeitspektrum ist nachteilhaft ein sogenanntes schlechtgestelltes Problem. Fehler in den Ausgangsdaten (Messwerte bzw. Werte der Masterkurve) können sich durch die Umrechnung vergrößern.

Mit Hilfe des Relaxationszeitspektrums und der verallgemeinerten Mischungsregel wird numerisch die Verteilung des Molekulargewichts m ermittelt, Die verallgemeinerte Mischungsregel beschreibt eine Beziehung zwischen dem Relaxationszeitspektrum h(m) und der Molekulargewichtsverteilung ω(m). Das Relaxationszeitspektrum h(m) steht in numerischer Form zur Verfügung. Deshalb wird diese Gleichung nach dem Stand der Technik numerisch gelöst.

Es ist Aufgabe der Erfindung, die Molekulargewichtsverteilung im Vergleich zum Stand der Technik verbessert ermitteln zu können.

Die Aufgabe der Erfindung wird gelöst, indem numerisch zur Verfügung stehende Ausgangsdaten G*(ω), also das Frequenz abhängig gemessene komplexes Schermodul oder die hieraus konstruierte Masterkurve), durch eine analytische Funktion beschrieben werden. Auf diese Weise steht in verbesserter Form eine genaue Kenntnis des Relaxationsverhaltens zur Verfügung und zwar vor allem auch bei längeren Relaxationszeiten. Dies ermöglicht eine zutreffende Ermittlung des Relaxationszeitspektrums und damit eine grundsätzlich zutreffendere Berechnung der Molekulargewichtsverteilung.

Aus dem Relaxationszeitspektrum und der verallgemeinerten Mischungsregel kann die Molekulargewichtsverteilung berechnet werden. Folgende allgemeine Mischungsregel kann für diese Berechnung verwendet werden:

$$w(m) = \frac{1}{\beta} \left( \frac{\alpha}{G_0} \right)^{\frac{1}{\beta}} h(m) \left[ \int_{\ln m}^{\infty} h(m')\, d\ln m' \right]^{\frac{1}{\beta} - 1}$$

mit
ω (m): Molekulargewichtsverteilung

m, m': Molekulargewicht

β : Mischungsparameter

Go: Plateaumodul

Der Mischungsparameter Beta (β) ist ein Anpassungsparameter, Die Praxis hat gezeigt, dass β gleich 2 ein gut geeigneter Wert ist, um die gesuchte, zutreffende Verteilungsfunktion aufzufinden, β kann aber beispielsweise auch 1 sein. Dieser Wert ist aber weniger gut geeignet.

Zunächst wird aus dem Frequenz abhängig gemessenem komplexen Schermodul in der Regel eine Masterkurve konstruiert. Hieraus wird ein Relaxationszeitspektrum h($_1$:) ermittelt und zwar vorzugsweise analytisch.

Um vom Relaxationszeitspektrum h($\tau$) zur Beziehung h(m) zu gelangen, wird in einer Ausführungsform von folgendem Zusammenhang zwischen Relaxationszeit $\tau$ und der molaren Masse m einer einzelnen Polymerkette ausgegangen, die erfahrungsgemäß den gesuchten Zusammenhang zwischen einem Molekulargewicht einer einzelnen Polymerkette und der Relaxationszeit $\tau$ zutreffend beschreibt:

$$\tau = k \cdot m^{\alpha}.$$

Das Molekulargewicht m einer einzelnen Polymerkette kann aus der Relaxationszeit $\tau$ berechnet werden und zwar durch die Beziehung $\tau = k \cdot m^{\alpha}$. Die Konstante k kann durch Kalibriermessungen ermittelt werden. Der Wert Alpha ($\alpha$) kann aus Messungen gewonnen werden. Zu diesem Zweck sind Polymere erhältlich, die über eine bekannte Molekulargewichtsverteilung verfügen. Solche Polymere werden Kolibrierungspolymere genannt, Werden die Relaxationszeiten bei einem solchen bekannten Polymer gemessen, so können so die gesuchten Konstanten ermittelt werden. So hat sich herausgestellt, dass regelmäßig $\alpha$=3,4 ist.

Es ist nicht erforderlich, die Konstante k zu ermitteln. Diese kann grundsätzlich auch beliebig gewählt werden. Die ermittelte Molekulargewichtsverteilung ist dann allerdings relativ und nicht absolut.

Eine Schwierigkeit bei der Auffindung einer analytischen Gleichung zur Beschreibung der numerisch zur Verfügung stehenden Ausgangsdaten besteht darin, den gesamten Bereich der Relaxation bei Polymeren zu beschreiben. Die gängigen, zur Verfügung stehenden Gleichungen erfassen nicht das viskose Fließen eines Polymers. Dies gilt insbesondere auch für die Cole - Cole Funktion

$$F_{cc}^{\star}(\omega) = F_0' \cdot \frac{(\imath\omega\tau_0)^b}{1 + (\imath\omega\tau_0)^b},$$

bekannt aus ,,Cole R., Cole H. J. Chem Phys" 9:341, 1941", die einen Relaxationsprozess mit der Stärke Fo, einer mittleren Relaxationszeit τ0 und einem Breitenparameter b beschreibt.

Um dieses Problem zu überwinden, wird in einer Ausführungsform der Erfindung eine Summe von Cole- Cole Funktionen mit einem Breitenparameter b=0,5 verwendet, um die numerisch zur Verfügung stehenden Ausgangsdaten durch eine analytische Funktion zu beschreiben. Es wurde festgestellt, dass die Summe aus wenigstens drei Cole- Cole Funktionen gebildet werden sollte, um zu einem guten Ergebnis zu gelangen. Nachfolgend wird die Summe von fünf Cole - Cole Funktionen abgebildet, die besonders zu bevorzugen ist.

$$F_{OCV}^{\star}(f) = \sum_{\nu=0}^{4} F_{0\nu} \frac{\sqrt{\imath \dfrac{f}{f_{0\nu}}}}{1 + \sqrt{\imath \dfrac{f}{f_{0\nu}}}},$$

wobei ω =2πf ist und τ0=1/(2πf0). Es hat sich unabhängig von der Zahl der aufsummierten Cole-Cole Funktionen herausgestellt, dass es besonders wichtig ist, den Breitenparameter 0,5 zu wählen, um zu einem besonders guten Ergebnis zu gelangen.

Die Parameter $F_{0v}$ und $f_{0v}$ sind vorteilhaft so zu wählen, dass die Summe der Cole-Cole-Funktionen folgendes ergibt: Der Realteil muss im Grenzfall kleiner Frequenzen in der doppelt logarithmischen Darstellung die Steigung 2 haben und der Imaginärteil im Grenzfall kleiner Frequenzen die Steigung 1. Das frequenzabhängige Verhalten des Speichermoduls und des Verlustmoduls im Bereich des viskosen Flusses wird dann korrekt beschrieben,

Im Fall einer Summe von fünf Cole-Cole Funktionen besteht demnach folgender Zusammenhang zwischen den vorgenannten Parametern $F_{0v}$ un d $f_{0v}$ :

$$f_{00} = f_0 \quad f_{01} = \frac{f_0}{\sqrt{\Delta}} \quad f_{02} = \frac{f_0}{\Delta} \quad f_{03} = \sqrt{\Delta} \cdot f_0 \quad f_{04} = \Delta \cdot f_0$$

$$F_{01} = F_{03} \;\; = \;\; F_0 \cdot \Delta^{\frac{1}{4}} \cdot \frac{(\Delta^2 - 1) \cdot (\Delta - 1)}{\Delta^{\frac{7}{2}} - \Delta^2 - \Delta^{\frac{3}{2}} + 1}$$

$$F_{02} = F_{04} \;\; = \;\; F_0 \cdot \Delta \cdot \frac{(\Delta - 1) \cdot (\Delta^{\frac{1}{2}} - 1)}{\Delta^{\frac{7}{2}} - \Delta^2 - \Delta^{\frac{3}{2}} + 1}$$

Werden diese drei Gleichungen erfüllt, so wird das frequenzabhängige Verhalten des Speichermoduls und des Verlustmoduls im Bereich des viskosen Flusses korrekt beschrieben.

Δ ist ein Anpassungsparameter und beschreibt den Frequenzabstand der diversen Prozesse, im gewissen Sinne handelt es sich um einen Breitenparameter, wie dieser von der Cole - Cole Funktion bekannt ist, Fo und fo sind weitere Anpassungsparameter. Insgesamt verbleiben damit drei Anpassungsparameter. Der oben genannte Grenzfall kleiner Frequenzen f liegt nun insbesondere dann vor, wenn f<<$f_0$/Δ.

Folgende Vorteile bietet das erfindungsgemäße Verfahren: Es können unlösliche Polymere sowie Polymere mit sehr hohem Molekulargewicht von mehr als 1.000,000 g/mol charakterisiert werden. Es können Langkettenverzweigung besser sichtbar gemacht werden.

Um die Molekulargewichtsverteilung mittels der Mischungsregel genauer zu berechnen, wird in einer Ausführungsform der Erfindung das Relaxationszeitspektrum um den Beitrag der Segmentrelaxationen des Glasprozesses bereinigt. In der Masterkurve wird der Glasübergang dadurch sichtbar, dass das Speichermodul von der Größenordnung 109 auf 106 absinkt. Eine präzise Unterscheidung von Glas-und Fließprozess Ist daher möglich. Der Glasprozess wird vorzugsweise analytisch durch eine eigenständige Funktion in vorgenannter Weise erfasst und anschließend herausgerechnet. Auch bei der Ermittlung des Glasprozessanteils im Relaxationsspektrum wird also erfindungsgemäß vorgegangen.

Zur Verdeutlichung der Vorteile und Möglichkeiten der vorliegenden Erfindung wurden analytisch ermittelte Molekulargewichtsverteilungen mit den durch GPC Messungen ermittelten Ergebnissen verglichen und zwar für drei Polymere NR, NBR und HNBR.

Es wurde weißer Kreppkautschuk (pale crepe, NR) für eine bestimmte Zyklenzahl (milling cycles) gemahlen. Alle zehn Zyklen wurde eine Probe genommen. Alle Mahlversuche wurden bei einer Temperatur von 20°C durchgeführt. Der Durchmesser der für das Mahlen eingesetzten Walzen betrug 200 mm. Die Drehfrequenz der langsameren Walze war auf 20 Umdrehungen/Minute eingestellt und auf 24 Umdrehungen/Minute für die schnellere Walze. Der Spalt zwischen den beiden Walzen betrug 0,35 mm. Eine grobe Näherung des Maximums der Scherrate γMAX kann nun gemäß abgeleitet werden,

$$\dot{\gamma}_{\mathrm{MAX}} = \frac{d\gamma_{\mathrm{MAX}}}{dt} = \frac{dx}{dt} \cdot \frac{1}{g} = v \cdot \frac{1}{g} = \frac{r \cdot \Delta\omega}{g} \approx 120 \left[\frac{1}{s}\right],$$

der Spalt g zwischen den Mahlwerken und die Differenz der Drehfrequenz der Walzen $\Delta\omega$ bekannt ist.

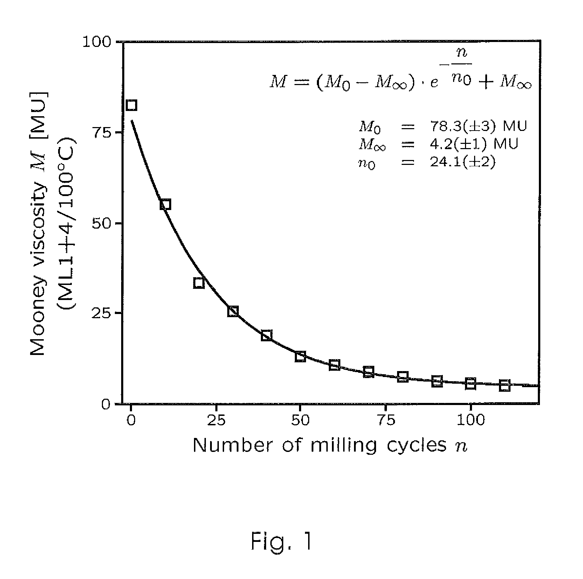

Fig. 1 zeigt den Einfluss der Anzahl der Mahlzyklen n auf die Mooney-Viskosität M (MU) (MH1+4/100C°). Die Mooney-Viskosität sinkt exponentiell bei steigender Anzahl der Mahlzyklen. Nach 100 Mahlzyklen bleibt die Mooney-Viskosität konstant bei einem Wert von ca, 4 Mooney-Graden, Die folgende Tabelle fasst die ermittelten Ergebnisse der Mooney- und GPC-Messungen als eine Funktion der Mahlzyklen zusammen.

| Zahl der Mahlzyklen | Mooney-Viskosität | $M_N$ [kg/mol] | $M_W$ [kg/mol] | $M_Z$ [kg/mol] | $M_P$ [kg/mol] | D |
|---|---|---|---|---|---|---|
| 0 | 83 | 311 | 892 | 1812 | 708 | 2,9 |
| 10 | 55 | 142 | 473 | 1178 | 452 | 3,3 |
| 20 | 33 | 110 | 302 | 550 | 357 | 2,8 |
| 30 | 25 | 104 | 263 | 465 | 292 | 2,5 |
| 40 | 19 | 96 | 229 | 401 | 236 | 2,4 |
| 50 | 13 | 91 | 201 | 348 | 200 | 2,2 |
| 60 | 11 | 77 | 172 | 304 | 157 | 2,2 |
| 70 | 9 | 73 | 159 | 276 | 147 | 2,2 |
| 80 | 7 | 76 | 155 | 263 | 152 | 2,1 |
| 90 | 6 | 73 | 146 | 246 | 141 | 2,0 |
| 100 | 5 | 68 | 141 | 240 | 134 | 2,1 |
| 110 | 5 | 65 | 133 | 226 | 124 | 2,0 |

$M_N$ ist das Zahlenmittel der Molmasse, Mw ist das Gewichtsmittel der Molmasse, $M_Z$ das Z-Mittel der Molmasse, $M_P$ der Spitzenwert der Molmassenverteilung und D die Polydispersität, In diesem Zusammenhang gilt: Die Gleichung

$$M^{(\nu)} = \frac{\sum_{i=1}^{N} N_i \cdot M_i^{\nu}}{\sum_{i=1}^{N} N_i \cdot M_i^{\nu-1}}$$

beschreibt eine allgemeine Beziehung für die Berechnung der durchschnittlichen molaren Masse eines Polymers bestehend aus N Ketten, $N_i$ bezeichnet die Zahl der Ketten mit der molaren Masse $M_i$.

Für V = 1 liefert diese Gleichung die Definition des Zahlenmittels der Molmasse $M_N$, V = 2 führt zur Definition des Gewichtsmittels der Molmasse Mw. V = 3 ist als Z-Mittel der Molmasse Mz bekannt.

Die Polydispersität D ergibt sich gemäß

$$D = \frac{M_W}{M_N} = N \cdot \frac{\sum\limits_{i=1}^{N} N_i \cdot M_i^2}{\left(\sum\limits_{i=1}^{N} N_i \cdot M_i\right)^2}$$

Die folgende Tabelle zeigt die ermittelten Ergebnisse der Mooney-und GPC-Messungen sämtlicher charakterisierten NBR-Proben. Die Reduzierung der Molmasse wurde durch eine Metathesereaktion erreicht, die eine statistische Reduzierung der Länge der Polymerketten ergibt. Die Gleichgewichtsbedingung der Metathesereaktion ist somit durch eine Polydispersität von 2 gekennzeichnet, Nach der Metathesereaktion wurden alle NBR-Proben zu HNBR hydriert (mit ungefähr 2% restlichen Doppelbindungen),

| Probe | Mooney Viskosität | $M_N$ [kg/mol] | $M_W$ [kg/mol] | $M_Z$ [kg/mol] | $M_P$ [kg/mol] | D |
|-------|-------------------|------------|------------|------------|------------|-----|
| NBR 1 | 28,8 | 82 | 262 | 875 | 112 | 3,2 |
| NBR 2 | 25,3 | 79 | 240 | 777 | 111 | 3,0 |
| NBR 3 | 21 | 75 | 201 | 551 | 108 | 2,7 |
| NBR 4 | 13,2 | 64 | 161 | 392 | 96 | 2,5 |
| NBR 5 | 8,1 | 56 | 135 | 307 | 87 | 2,4 |
| NBR 6 | 5,9 | 53 | 117 | 247 | 82 | 2,2 |
| NBR 7 | 2,7 | 44 | 89 | 164 | 72 | 2,0 |

Die folgende Tabelle zeigt die Ergebnisse der Mooney- und GPC-Messungen. Die NBR- und die HNBR-Proben mit einer identischen Nummer (z.B. NBR 1 und HNBR 1) bezeichnen das identische Polymer vor und nach dem Hydrierungsprozess. Jeder Unterschied bei den Eigenschaften kann daher auf den Hydrierungsprozess zurückgeführt werden.

| Probe | Mooney Viskosität | $M_N$ [kg/mol] | $M_W$ [kg/mol] | $M_Z$ [kg/mol] | $M_P$ [kg/mol] | D |
|-------|-------------------|------------|------------|------------|------------|-----|
| HNBR 1 | 76,8 | 96 | 278 | 764 | 131 | 2,9 |
| HNBR 2 | 66,9 | 88 | 260 | 711 | 127 | 2,9 |
| HNBR 3 | 58,6 | 87 | 238 | 627 | 125 | 2,7 |
| HNBR 4 | 40,2 | 81 | 193 | 457 | 112 | 2,4 |
| HNBR 5 | 29,6 | 67 | 159 | 342 | 103 | 2,4 |
| HNBR 6 | 22,9 | 63 | 143 | 299 | 98 | 2,3 |
| HNBR 7 | 11,6 | 52 | 107 | 201 | 83 | 2,1 |

Das Relaxationsverhalten sämtlicher Proben wurde durch Masterkurven charakterisiert, die aus Frequenzdurchläufen des komplexen Schermoduls G*(ω) bei verschiedenen Temperaturen konstruiert wurden. Im Temperaturbereich von -80°C bis 100°C wurden die Frequenzdurchläufe des komplexen Moduls mit dem Mettler STDA 861e im Bereich von 0,01 Hz bis 1000 Hz unter Verwendung des "Doppelsandwich-Probenhalters" gemessen. Das Rheometer MCR 300 von Paar Physica wurde für frequenzabhängige Messungen von 0,001 Hz bis 100 Hz im Temperaturbereich von 40°C bis 140°C und für Kriechmessungen für Zeiten, die von 0,1 s bis 40000 s bei 100°C reichten, verwendet.
Kriechmessungen werden normalerweise verwendet, um das zeitabhängige dynamische-mechanische Verhalten für lange Zeiten oder kleine Frequenzen († ~ 1/f) zu charakterisieren. Mit dem Programm "NLREG" (Freiburg, Material Research Center. NLREG for nonlinear regularization, Version Rheology 2.0, 2001) wurde der frequenzabhängige Modul $G^*(\omega)$ aus dem zeitabhängigen Kriechmodul $G(t)$ berechnet. Dies ermöglicht die Charakterisierung des frequenzabhängigen Verhalten bei sehr kleinen Frequenzen ($f < 10^{-4}$ Hz),

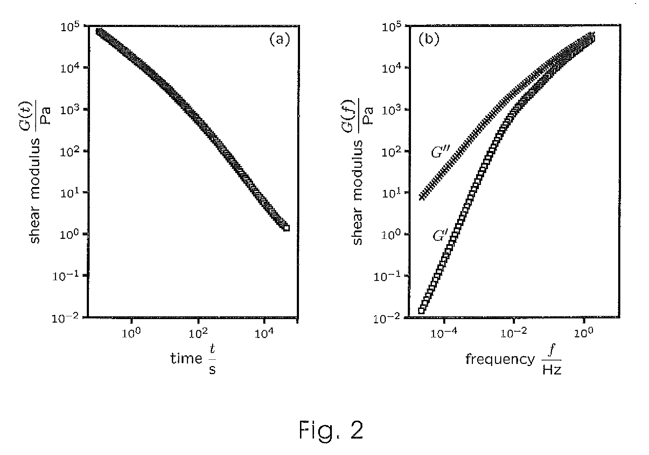

Fig. 2 zeigt das Ergebnis der Berechnung des frequenzabhängigen Verhaltens aus einem Kriechversuch für Naturkautschuk (weißer Kreppkautschuk), der 60 Zyklen gemahlen wurde, Gezeigt wird eine Kriechmessung (Fig. 2a) und ein berechnetes frequenzabhängiges Verhalten (Fig. 2b) für NR (weißer Kreppkautschuk) nach 60 Mahlzyklen.

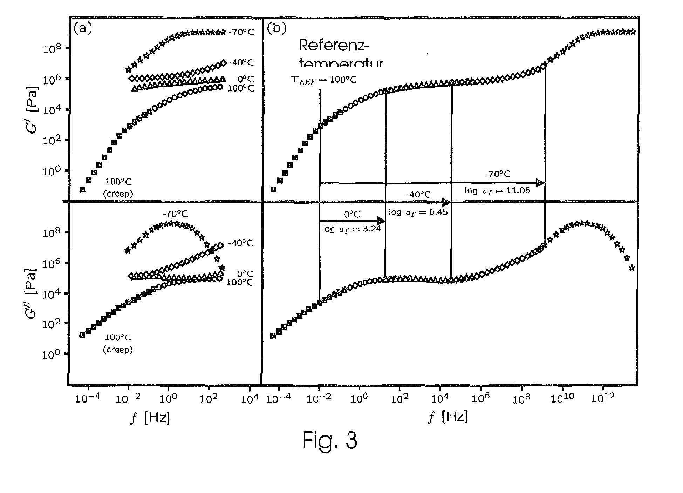

Unter Verwendung des Zeit-Temperatur-Äquivalenzprinzips wurden die berechneten frequenzabhängigen Daten aus Kriechversuchen und die frequenzabhängigen Messungen bei verschiedenen Temperaturen zu einer Masterkurve, die das dynamisch-mechanische Verkombiniert. Fig. 3 zeigt das Ergebnis der Konstruktion der Masterkurve für ein NR nach 60 Mahlzyklen. Gezeigt werden frequenzabhängige Messungen (Fig. 3a) und konstruierte Masterkurve (Fig. 3b) für NR (weißer Kreppkautschuk) nach 60 Mahlzyklen,

Um die Messwerte analytisch auszuwerten, wurde eine Summe von fünf Cole-Cole-Funktionen mit einem konstanten b=0,5 verwendet:

$$F_{CCV}^{\star}(f) = \sum_{\nu=0}^{4} F_{0\nu} \frac{\sqrt{\imath \frac{f}{f_{0\nu}}}}{1 + \sqrt{\imath \frac{f}{f_{0\nu}}}}$$

Die Parameter $F_\nu$ und $f_{0\nu}$ wurden so gewählt, dass die folgenden Randbedingungen erfüllt wurden:

$$f_{00} = f_0 \quad f_{01} = \frac{f_0}{\sqrt{\Delta}} \quad f_{02} = \frac{f_0}{\Delta} \quad f_{03} = \sqrt{\Delta} \cdot f_0 \quad f_{04} = \Delta \cdot f_0$$

$$F_{01} = F_{03} \quad = \quad F_0 \cdot \Delta^{\frac{1}{4}} \cdot \frac{(\Delta^2 - 1) \cdot (\Delta - 1)}{\Delta^{\frac{7}{2}} - \Delta^2 - \Delta^{\frac{3}{2}} + 1}$$

$$F_{02} = F_{04} \quad = \quad F_0 \cdot \Delta \cdot \frac{(\Delta - 1) \cdot (\Delta^{\frac{1}{2}} - 1)}{\Delta^{\frac{7}{2}} - \Delta^2 - \Delta^{\frac{3}{2}} + 1}$$

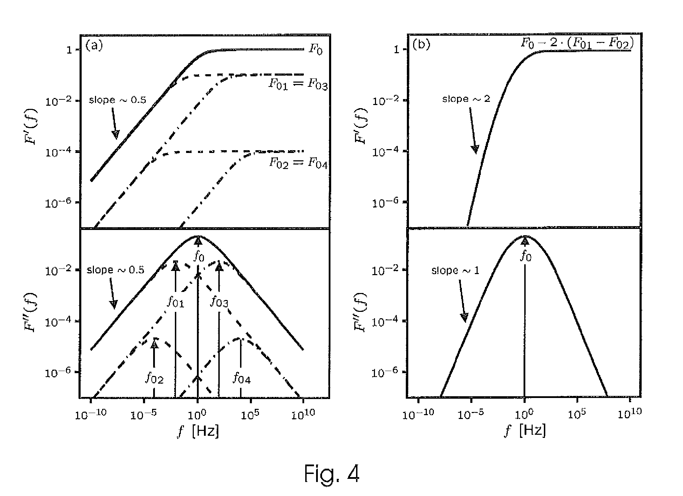

Diese Cole-Cole-Funktion mit viskoser Beendigung $F_{CCV}^*(f)$ hängt nun nur von den Parametern $F_0$, fo und $\Delta$ ab. Fig. 4 zeigt ein Beispiel für einen Relaxationsprozess mit viskoser Beendigung. In Fig. 4a sind die fünf Cole-Cole-Funktionen separat aufgetragen, Fig. 4b zeigt den resultierenden Prozess mit Fo = 1, fo = 1 und $\Delta$ = 100.

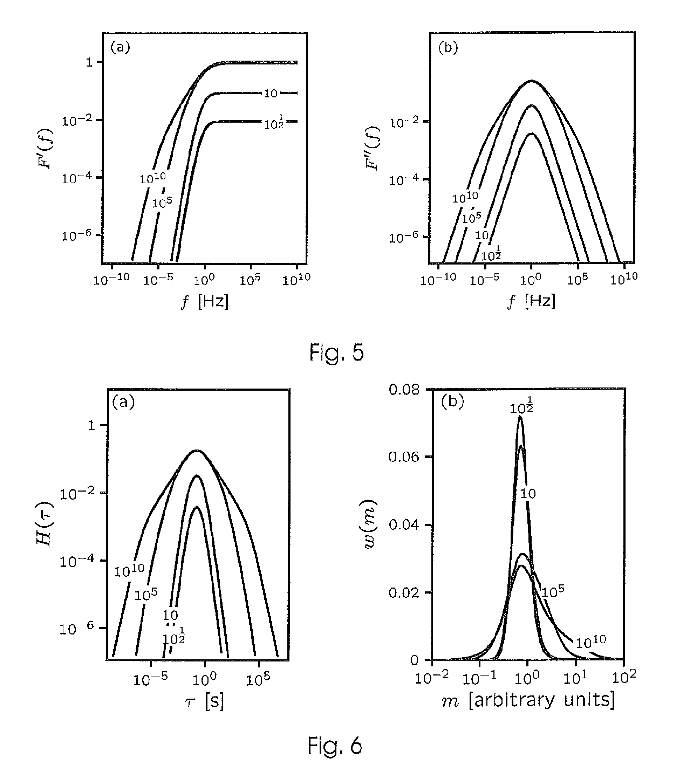

Figur 5 verdeutlicht den Einfluss des Parameters $\Delta$ ($\Delta = 10^{10}$, $10^5$, 1 und $10^{1/2}$) auf das frequenzabhängige Verhalten. Die Einführung der Beendigungsbedingung ergibt die folgenden Eigenschaften des Relaxationsprozesses:

- Unter den Bedingungen der viskosen Strömung werden die Speicher- und Verlustmodule durch ein frequenzabhängiges Verhalten charakterisiert, das das Verhalten einer idealen Newtonschen Flüssigkeit widerspiegelt,
- Die Frequenz, bei der der Verlustmodul sein Maximum erreicht, ist identisch mit der Relaxationsfrequenz $f_0$.
- Der Relaxationsprozess mit viskoser Beendigung wird durch drei Parameter charakterisiert: $F_0$, $f_0$ und $\Delta$. Der Breitenparameter b ist konstant (b = 0,5).

Das Spektrum der Relaxationszeiten wird analytisch auf der Basis der Gleichungen

$$F_{cc}(\tau) = \frac{F_0}{\pi} \frac{\sin\left((1-b)\,\pi\right)\cdot\left(\frac{\tau}{\tau_0}\right)^b}{1 - 2\cos\left((1-b)\,\pi\right)\cdot\left(\frac{\tau}{\tau_0}\right)^b + \left(\frac{\tau}{\tau_0}\right)^{2b}}$$

und

$$F^\star_{CCV}(f) = \sum_{\nu=0}^{4} F_{0\nu} \frac{\sqrt{i\,\frac{f}{f_{0\nu}}}}{1 + \sqrt{i\,\frac{f}{f_{0\nu}}}}$$

berechnet. Das Ergebnis wird in Fig. 6a als eine Funktion des Parameters $\Delta$ gezeigt. Fig. 6b zeigt das Ergebnis der Berechnung der Verteilung der Molmasse unter Verwendung der Cole-Cole Gleichung

$$w(m) = \frac{\alpha}{\beta}\left(\frac{1}{G_0}\right)^{\frac{1}{\beta}} \frac{\sum_i \dfrac{F_{0i}}{\pi} \dfrac{\sin\left((1-b_i)\,\pi\right)\cdot\left(\frac{m}{m_{0i}}\right)^{b_i\cdot\alpha}}{1 - 2\cos\left((1-b_i)\,\pi\right)\cdot\left(\frac{m}{m_{0i}}\right)^{b_i\cdot\alpha} + \left(\frac{m}{m_{0i}}\right)^{2b_i\cdot\alpha}}}{\left(\sum_i \dfrac{F_{0i}}{\pi b_i}\cdot\left\{\dfrac{b_i\pi}{2} - \arctan\left[\dfrac{1 - \left(\frac{m_{0i}}{m}\right)^{b_i\cdot\alpha}}{1 + \left(\frac{m_{0i}}{m}\right)^{b_i\cdot\alpha}}\cdot\tan\dfrac{b_i\pi}{2}\right]\right\}\right)^{1-\frac{1}{\beta}}}$$

für die obigen Relaxationsprozesse.

Im folgenden Abschnitt wird die Berechnung der Molmassenverteilung nach dem erfindungsgemäßen Verfahren, nachfolgend DMA genannt, für NR nach 60 Mahlzyklen demonstriert. Figur 6 verdeutlicht den Einfluss des Beendigungsverhältnisses ($\Delta = 10^{10}$, $10^5$, 1 und $10^{1/2}$) auf das Spektrum der Relaxationszeiten.

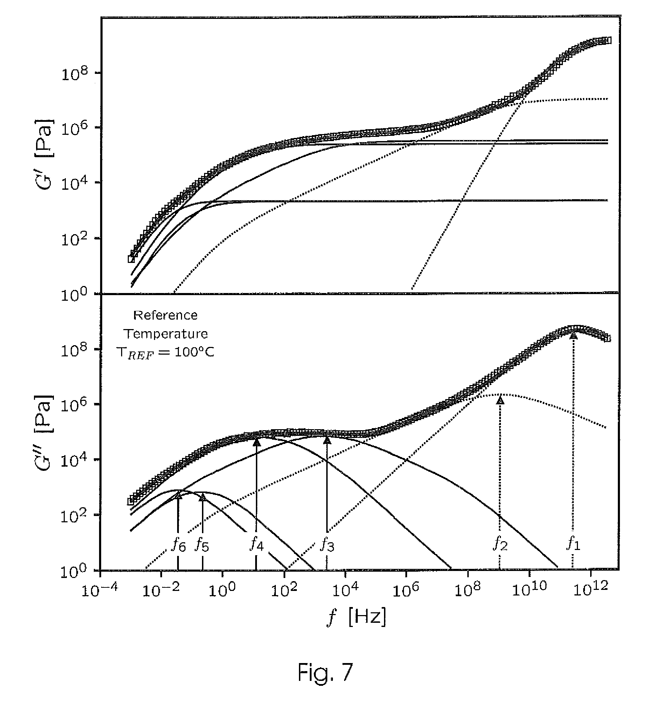

Die Masterkurve (siehe Fig. 3a) wurde analytisch durch eine Summe von sechs Relaxationsfunktionen mit viskoser Beendigung beschrieben, Fig. 7 zeigt das Ergebnis einer Anpassung mit der Fehlerquadratmethode der Relaxationsfunktionen auf die frequenzabhängigen Werte der Speicher- und Verlustmodulen,

Die Anpassung mit der Fehlerquadratmethode wurde als Excel-Makro implementiert und erlaubt eine Anpassung von bis zu 8 Relaxationsfunktionen mit viskoser Beendigung an die Werte der Speicher- und Verlustmodulin einer gegebenen Masterkurve. Die beste Anpassungsbedingung wird in folgender Gleichung gezeigt.

$$\sum_{i=1}^{N}\left[\left(\log G'(f_i) - \log\sum_\nu F'_{CCV\nu}(f_i)\right)^2 + \left(\log G''(f_i) - \log\sum_\nu F''_{CCV\nu}(f_i)\right)^2\right] \overset{!}{=} \mathrm{MIN}$$

Aufgrund der starken Schwankung der Speicher- und Verlustmodulin wurde eine logarithmische Skalierung der Daten und Funktionswerte gewählt. Dies beschränkt auch die Dominanz der Bedingungen der Glasübergangs über den gesamten Frequenzbereich.

Eine Anpassung der Relaxationsfunktionen an die Masterkurve (siehe Fig. 7) ergibt die Parameter der Relaxationsfunktionen. Figur 7 zeigt eine analytische Beschreibung der Masterkurve von NR (weißer Kreppkautschuk) nach 60 Mahlzyklen mit viskos beendeten Relaxationsfunktionen.

Da das Spektrum von Relaxationszeiten durch eine Summe von Relaxationsfunktionen mit viskoser Beendigung beschrieben wird (siehe Gleichung

$$F_{cc}(\tau) = \frac{F_0}{\pi} \frac{\sin\left((1-b)\,\pi\right)\cdot\left(\frac{\tau}{\tau_0}\right)^b}{1 - 2\cos\left((1-b)\,\pi\right)\cdot\left(\frac{\tau}{\tau_0}\right)^b + \left(\frac{\tau}{\tau_0}\right)^{2b}}),$$

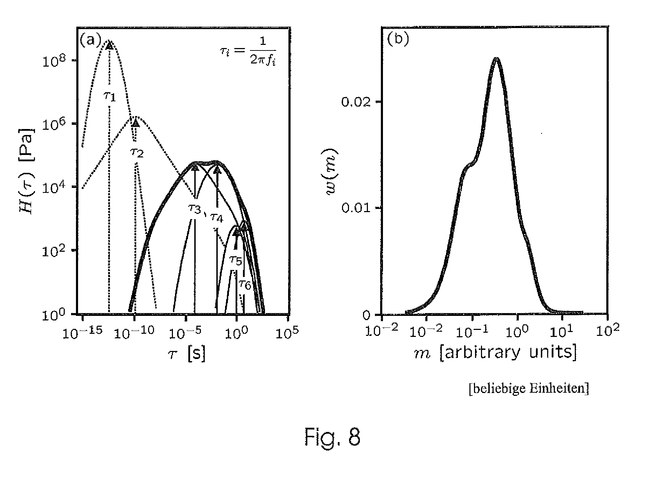

ist eine numerische Berechnung des Spektrums von Relaxationszeiten nicht mehr notwendig. Fig. 8 zeigt das Spektrum von Relaxationszeiten, das aus den Parametern der Relaxationszeiten berechnet wurde.

Bei der Berechnung der Molmassenverteilung aus dem Spektrum der Relaxationszeiten wurde von folgender Annahme ausgegangen. Thimm et al. (Thimm W., Friedrich C., Honerkamp J., J. Rheol., 6:43, 1999) bemerkten, dass nur die Zeit einer Reptation einer Kette in einer Röhre direkt mit der Kettenlänge korreliert. Kürzere Relaxationszeiten werden durch die Relaxation von Kettensegmenten (den sogenannten Rouse-Moden) verursacht. Eine Berechnung der Verteilung der Kettenlänge erfolgt daher vorteilhaft durch Subtraktion der Rouse-Moden von dem Spektrum der Relaxationszeiten.

Da die theoretische Ableitung des Beitrags der Rouse-Moden (Rouse, P. E., J. Chem, Phys., 21;1272, 1953) zu dem Spektrum der Relaxationszeiten nicht gut mit den Versuchsergebnissen übereinstimmt (insbesondere unter den Bedingungen des Glasübergangs), wurde ein empirischerer Ansatz gewählt und die Relaxation von Kettensegmenten wurde durch zwei Relaxationsfunktionen mit viskoser Beendigung angenähert (siehe gestrichelte Linien in Fig. 8a). Nach der Subtraktion der beiden Relaxationsfunktinen wurde die Verteilung der Molmasse analytisch aus dem Spektrum der verbliebenen Relaxationsfunktionen berechnet (sieh Fig. 8b). Figur 8 zeigt ein Spektrum der Relaxationszeit und Verteilung der Molmasse von NR (weißer Kreppkautschuk) nach 60 Mahlzyklen,

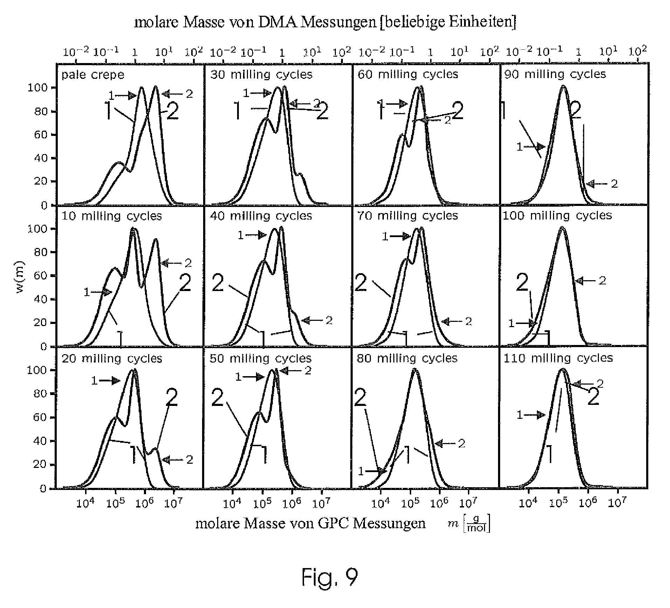

Fig. 9 zeigt das Ergebnis der GPC (Kurven 1) und der DMA (Kurven 2) von NR als eine Funktion der Anzahl der Mahlzyklen gemäß der ersten Tabelle. Da die dynamisch-mechanischen Daten, also die DMA - Daten nicht kalibriert wurden, sind die Werte für die Molmasse auf den oberen Achsen nur relative Zahlen.

Der Vergleich der Molmassenverteilung aus GPC und DMA zeigt eine gute Korrelation für die NR-Proben, die für mehr als 80 Zyklen gemahlen wurden, und erlaubt eine grobe Kalibrierung der dynamisch-mechanischen Daten. Für kleinere Mischzyklenzahlen unterscheiden sich die Ergebnisse beider Verfahren signifikant.

Die Ergebnisse der dynamisch-mechanischen Analyse können wie folgt zusammengefasst werden.

- Das ungemahlene NR wird durch eine bimodale Verteilung mit den Molmassen $M_1 \approx 10^5$ g/mol und $M_2 \approx 2 \times 10^6$ g/mol gekennzeichnet.
- Nach 10 Mahlzyklen erscheint eine dritte Spitze ($M_3 \approx 4 \times 10^5$ g/mol) in der Verteilung der Molmasse, Die Molmasse dieser Spitze bleibt unverändert, bis 50 Mahlzyklen durchgeführt wurden.
- Die Intensität der Spitze mit der höchsten Molmasse $M_2 \approx 2 \times 10^6$ g/mol verringert sich mit einer wachsenden Anzahl von Mahlzyklen, und verschwindet nach ungefähr 50 Mischzyklen,
- Nach ungefähr 50 Mahlzyklen ist die Molmassenverteilung wiederum bimodal mit $M_1 \approx 7 \times 10^4$ g/mol und $M_3 \approx 3 \times 10^5$ g/mol. Ein weiteres Anwachsen der Anzahl der Mahlzyklen verringert die Intensität der Fraktion mit der größeren Molmasse $M_3$. Bei ungefähr 80 Mahlzyklen liegt die Verteilung der Molmasse nahe an einer monomodalen Verteilungsfunktion mit einem Maximum von ungefähr $M_P \approx 1,4 \times 10^6$ g/mol.
- Eine weitere Steigerung der Anzahl der Mahlzyklen ergibt nur eine geringe Reduzierung des Spitzenwertes (von ungefähr $1,4 \times 10^5$ g/mol nach 80 Mahlzyklen bis auf ungefähr $1,0 \times 10^5$ g/mol nach 110 Mahlzyklen).

Die Ergebnisse der dynamisch-mechanischen Analyse (DMA) können interpretiert werden, wenn zwei Annahmen gemacht werden.

1. Das untersuchte NR ist ein Blend von mindestens drei Fraktionen mit verschiedenen Molmassen, wobei die Fraktion mit der höchsten Molmasse dominant ist.

2. Kürzere Ketten sind stabiler als längere Ketten

Wenn die längeren Ketten zuerst aufgebrochen werden, wird die Fraktion mit der größten Molmasse zunächst am meisten durch den Mahlprozess beeinflusst werden.

Nach 10 Mahlzyklen dominiert die Fraktion mit der höchsten Molmasse nicht länger die Mischung. Nach ungefähr 50 Mahlzyklen sind die längsten Ketten beinah verschwunden. Die Verteilung der Molmasse wird nun durch die Fraktion mit der zweitgrößten Molmasse dominiert, Nach 80 Mahlzyklen sind die meisten Ketten aufgebrochen, und die Molmassenverteilung wird durch die Fraktion mit der geringsten Molmasse dominiert.

Falls die Stabilität einer Kette von ihrer Länge abhängt, wird die Verringerung der Molmasse auf einen kritischen Wert beschränkt. Bei der kritischen Länge ist das angelegte mechanische Feld, das proportional zur Scherrate ist (siehe Gleichung

$$\dot{\gamma}_{\text{MAX}} = \frac{d\gamma_{\text{MAX}}}{dt} = \frac{dx}{dt} \cdot \frac{1}{g} = v \cdot \frac{1}{g} = \frac{r \cdot \Delta\omega}{g} \approx 120 \left[\frac{1}{s}\right]_,$$

zu klein, um die Kette aufzubrechen, Dies weist auf eine im Wesentlichen konstante Molmassenverteilung nach einer bestimmten Anzahl von Mahlzyklen hin. Bei dem durchgeführten Versuch beträgt der Maximalwert der Molmasse ungefähr $1 \times 10^5$ g/mol nach 90 Mahlzyklen, und bleibt konstant nach weiterem Mahlen.

Der Vergleich der Ergebnisse aus dem DMA mit den Ergebnissen der GPC-Analyse zeigt signifikante Unterschiede für die ersten Mahlzyklen. Ein Anstieg der Anzahl der Mahlzyklen führt zu einer Vergleichbarkeit der beiden Verfahren.

Der Unterschied bei den Ergebnissen der beiden Verfahren kann erklärt werden, wenn bedacht wird, dass die Fraktion mit der großen Molmasse unlöslich ist, und daher nicht durch das GPC-Verfahren detektiert wird. Nach einer gewissen Anzahl von Mahlzyklen ist die unlösliche Fraktion mit der größten Molmasse entfernt worden, Die Ergebnisse der GPC-Messung ähneln daher dann den nach der Erfindung ermittelten Ergebnissen (DMA). Ein quantitativer Vergleich der beiden Verfahren zeigt, dass die Anwendung des GPC-Verfahrens problematisch ist, wenn die Molmasse der Ketten in dem Polymer $1 \times 10^6$ g/mol überschreitet. Ketten mit einer größeren Molmasse werden den Ergebnissen nach zu urteilen nicht durch das verwendete GPC-Verfahren detektiert,

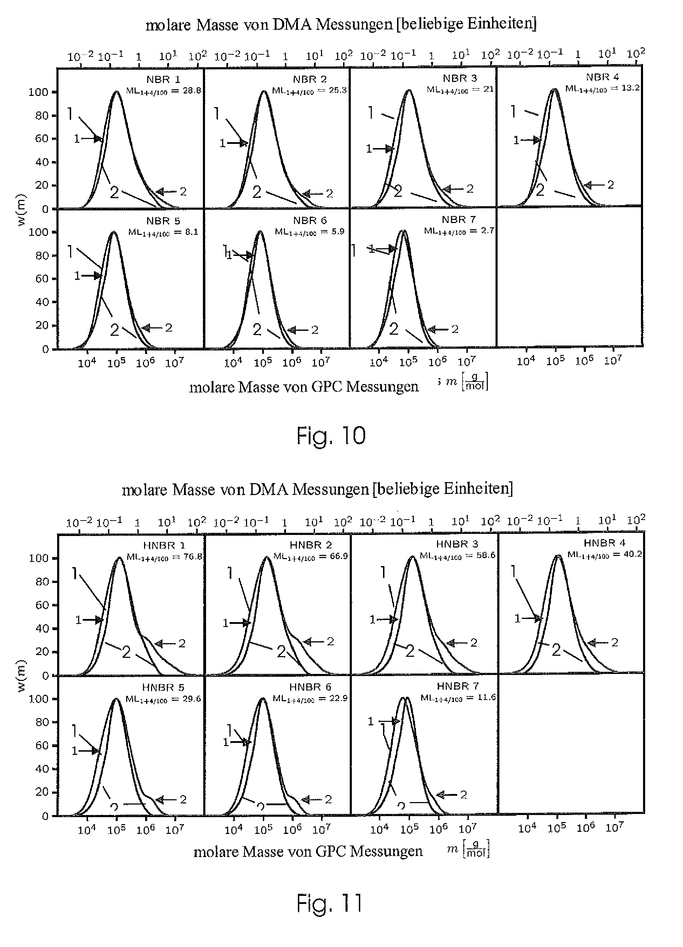

Die Ergebnisse der Bestimmung der Molmassenverteilung von NBR-und HNBR-Proben werden in den Figuren 10 und 11 gezeigt. Die Kurven 1 beziehen sich auf die Ergebnisse der GPC-Messungen und die Kurven 2 auf die Ergebnisse der dynamisch-mechanischen Versuche (DMA), also auf die nach der Erfindung erhaltenen Ergebnisse. Ein quantitativer Vergleich beider Verfahren ist wiederum nicht möglich, da die Ergebnisse der dynamisch-mechanischen Messungen (DMA) nicht unabhängig kalibriert wurden. Somit sind die Werte der Molmasse aus der DMA auf den oberen Achsen wieder relative Zahlen.

Ein Vergleich der NBR-Ergebnisse zeigt eine gute qualitative Übereinstimmung zwischen dem GPC- und dem DMA-Verfahren. Signifikante Unterschiede treten nur bei höheren Molmassen auf. Der Unterschied zwischen den Ergebnissen der beiden Verfahren kann wiederum nur mit unlöslichen Polymerfraktionen erklärt werden, Diese unlöslichen Fraktionen, die nicht mit dem GPC-Verfahren detektiert werden können, könnten entweder durch Ketten mit einer sehr hohen Molmasse, oder durch Ketten mit einer verzweigten Struktur verursacht werden,

Die Theorie hinter beiden Verfahren basiert auf der Annahme, dass alle Ketten in dem Polymer linear sind. Wenn eine Verzweigung auftritt, werden die Ergebnisse der GPC und der DMA auf verschiedene Weise beeinflusst.

Das Prinzip des GPC-Verfahrens ist die Abhängigkeit der Größe einer Polymerkette (d.h., der Trägheitsradius) von der Diffusion innerhalb poröser Materialien. Eine kleinere Kette benötigt eine längere Zeit, um durch ein poröses Material zu diffundieren, als eine längere Kette. Da verzweigte Strukturen immer einen kleinere Trägheitsradius aufweisen als lineare Ketten vergleichbarer Molmasse, benötigt die verzweigte Struktur länger für den Diffusionsprozess. Die Molmasse einer verzweigten Struktur wird daher durch die GPC-Messung unterschätzt.

Figur 10 zeigt eine Charakterisierung der Molmasse von NBR mit GPC (Kurven 1) und DMA (Kurven 2). Bei der DMA ist zu bedenken: Da die Molmasse in Beziehung mit der Relaxationszeit der gesamten Kette steht und die Relaxation einer verzweigten Kette langsamer ist als die Relaxation einer lineare Kette mit vergleichbarer Molmasse, kann die Molmasse durch die DMA überschätzt werden. Ein Vergleich der Molmassenverteilung aus GPC und aus DMA im Bereich der großen Molmassen kann daher als ein Hinweis für die Anwesenheit der Verzweigung langer Ketten verwendet werden.

Wenn die Molmassenverteilungen aus GPC und DMA für die NBR-Proben in der Region größerer Molmassen verglichen werden (siehe Fig. 10), sind die DMA-Werte geringfügig höher als die Werte der GPC-Messungen. Dies weist auf die Existenz eines kleinen Beitrags verzweigter Strukturen langer Ketten hin.

Wenn derselbe Vergleich für die HNBR-Proben durchgeführt wird (siehe Fig. 11 - Charakterisierung der Molmassenverteilung von HNBR mit GPC (Kurven 1) und DMA (Kurven 2)), zeigen die Ergebnisse aus der DMA eine signifikant höhere Fraktion von Ketten mit höheren Molmassen als die Ergebnisse aus den GPC-Messungen. Eine mögliche Erklärung wäre eine zusätzliche Verzweigung der Polymerketten während der Hydrierung.

Eine alternative Erklärung basiert auf dem Einfluss der Flexibilität der Polymerketten auf deren Relaxationsverhalten. Die gesteigerte Flexibilität einer hydrierten Polymerkette ergibt eine größere Relaxationszeit, die eine erhöhte Volumenviskosität verursacht. Da der Einfluss der Kettenflexibilität auf die Viskosität für verzweigte Ketten langer Ketten stärker ist, würde der Beitrag verzweigter Ketten zu der Molmassenverteilung vergrößert, wenn die verzweigten Ketten mehr Flexibilität gewinnen.

Auf der Basis der vorliegenden Ergebnisse ist eine quantitative Validierung einer möglichen Verzweigungsreaktion während der Hydrierungreaktion nicht möglich, Dies würde eine Wiederholung der Versuche mit einer Serie von NBR-Proben mit unterschiedlicher Viskosität und vollständiger linearer Kettenstruktur notwendig machen.

Wenn während der Hydrierung keine Nebenreaktion auftritt, würde die Hydrierung nur die Flexibilität der Polymerketten beeinflussen, Da der Einfluss der Kettenflexibilität auf die Viskosität von der Kettenstruktur abhängt, und die Hydrierung einer verzweigten Struktur eine signifikant höhere Viskosität ergibt, als die Hydrierung eine linearen Kettenstruktur, könnten die Unterschiede zwischen den NBR- und den HNBR-Ergebnissen ein qualitativer Hinweis für die Existenz einer Fraktion verzweigter Polymerketten in dem NBR sein.

Ein häufig diskutierter Effekt ist der sogenannte "Mooney-Sprung", der die Tatsache beschreibt, dass die Mooney-Viskosität nach der Hydrierung der Doppelbindungen des NBR signifikant ansteigt.

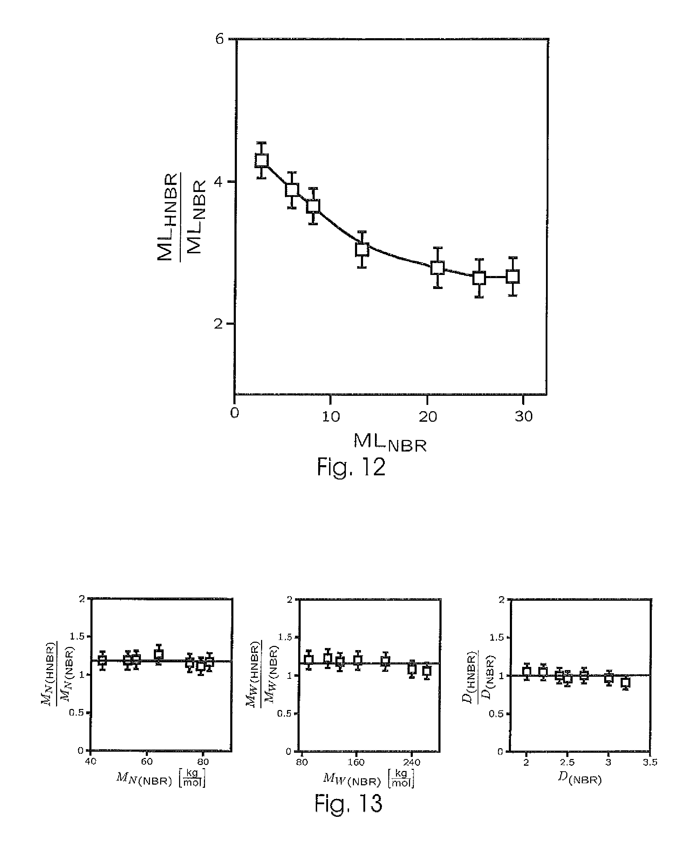

Dies wird in Fig. 12 deutlich gezeigt, wo die Mooney-Viskositäten ML1+4/100°C(im folgenden ML genannt) des NBR gegen das Verhältnis des ML des HNBR und NBR aufgetragen sind. Der ML des HNBR ist nicht nur größer als der des NBR, von dem es abgeleitet wurde, sondern das Verhältnis der Mooney-Viskosität erreicht die größten Werte, wenn die Mooney-Viskosität des NBR gering ist, Je höher die Mooney-Viskosität des NBR, desto geringer der Effekt des "Mooney-Sprungs",

Bisher ist nicht klar, welcher Mechanismus für den "Mooney-Sprung" und seiner Abhängigkeit auf die Mooney-Viskosität des NBR verantwortlich ist.

Eine mögliche Erklärung für die höheren Mooney-Viskositäten des HNBR im Vergleich zu dem NBR ist die Existenz von chemischen Nebenreaktionen während des Hydrierungprozesses, die eine höhere Menge von hochmolekularen und/oder verzweigten Ketten erzeugen.

Eine alternative Erklärung basiert auf der Flexibilität der NBR-Polymerketten, die wegen der Hydrierung ansteigt. Die höhere Flexibilität der hydrierten Polymerketten würde eine höhere Menge an Verhakungen ergeben. Da eine größere Anzahl von Verhakungen in höherer Viskosität resultiert, kann geschlossen werden, dass eine erhöhte Flexibilität der Hauptkette immer von einer höheren Volumenviskosität begleitet wird.

Ein Vergleich der GPC-Ergebnisse der NBR- und HNBR-Polymere zeigt, dass der Zahlen- und Gewichtmittelwert der Molmassen der HNBR-Proben nur ungefähr 10% bis 20% höher sind, als die Durchschnittswerte der entsprechenden NBR-Proben (siehe Fig. 13 - Vergleich der Durchschnittswerte der Molmassen von NBR und HNBR, gemessen mit GPC). Des Weiteren zeigen die GPC-Ergebnisse keine Abhängigkeit des Verhältnisses der Molmassen von der Molmasse des NBR.

Da die GPC nicht in der Lage ist, sehr hochmolekulare und/oder langkettig verzweigte Fraktionen zu detektieren, können die Ergebnisse des DMA genutzt werden, um den Mechanismus hinter dem Mooney-Sprung aufzuklären,

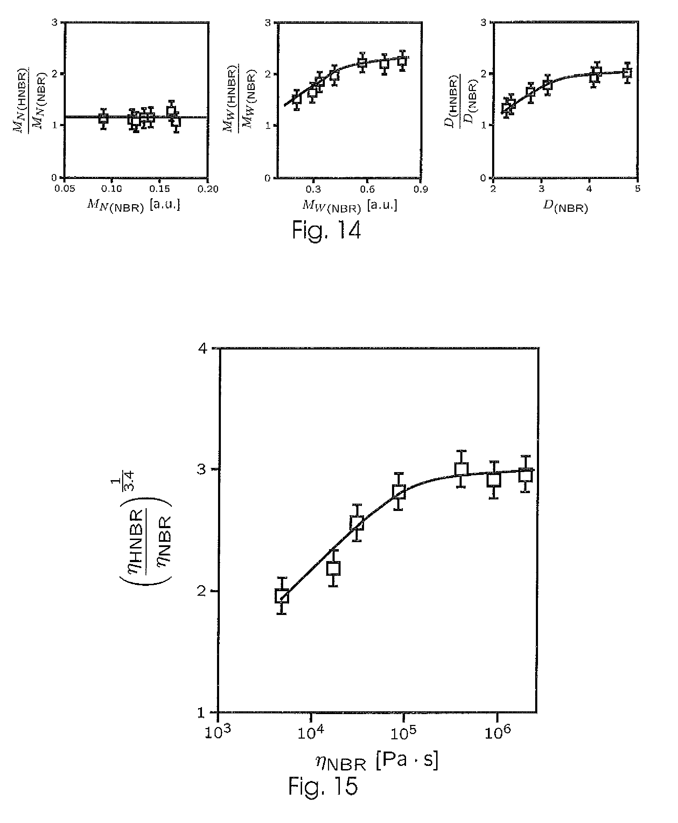

Ein Vergleich der dynamisch-mechanischen Ergebnisse (siehe Fig. 14) der NBR- und HNBR-Polymere zeigt, dass der Zahlenmittelwert der Molmasse des HNBR ungefähr 10% bis 20% höher ist als die Durchschnittswerte der entsprechenden NBR-Proben, Das Resultat ähnelt den Ergebnissen der GPC-Messungen.

Die Charakterisierung der Gewichtsmittelwerte der Molmassen ergibt entgegengesetzte Ergebnisse für die GPC- und DMA-Messung. Während die GPC-Ergebnisse ein konstantes Verhältnis zwischen dem Gewichtmittelwert der Molmassen von HNBR und NBR detektiert, detektiert die DMA einen deutlichen Einfluss der Molmasse, Das Verhältnis des Gewichtsmittelwertes der Molmasse von HNBR und NBR steigt mit der Molmasse an und erreicht einen Plateauwert für die höchsten Molmassen (siehe das mittlere und das rechte Diagramm in Fig. 14 - Vergleich der Durchschnittswerte der Molmassen von NBR und HNBR, gemessen mit dynamisch-mechanischer Analyse).

Die Ergebnisse der GPC und der dynamisch-mechanischen Analyse (DMA) von NBR und HNBR deuten zwei mögliche Mechanismen als Grund für den Mooney-Sprung an.

- Die höhere Flexibilität der hydrierten NBR-Ketten
  Dies ergibt einen vergrößerten Trägheitsradius und eine höhere Viskosität. Da die Molmasse aus dem Trägheitsradius (GPC-Verfahren) oder aus der Volumenviskosität (DMA) berechnet wird, würde eine Steigerung der Flexibilität der hydrierten Kette einen konstanten Anstieg der Molmasse verursachen. Die experimentelle Bestätigung ist durch die Tatsache gegeben, dass das Verhältnis des Zahlenmittelwertes der Molmasse für beide Verfahren unabhängig von der Molmasse zu sein scheint,

- Die Existenz von langkettig verzweigten Ketten
  Wenn der Verzweigungsgrad vergrößert wird, schrumpft der Trägheitsradius, und die Viskosität vergrößert sich. Daher unterschätzt das GPC-Verfahren die Molmasse einer verzeigten Struktur, während die dynamisch-mechanische Analyse der entgegengesetzten Effekt ergibt.
  Da die Hydrierung eine höhere Kettenflexibilität ergibt, ist der Einfluss der Verzweigung auf den Trägheitsradius und die Viskosität größer bei einer flexibleren Kette.
  Unterschiede in den Ergebnissen des GPC- und des dynamisch-mechanischen Verfahrens bei höheren Molmassen könnten daher einer Fraktion von verzweigten Polymerketten zugeschrieben werden. Dies kann man in den Diagrammen der Molmassenverteilung (siehe Fig. 10 und Fig. 11) und im Vergleich des Einflusses der Molmasse auf den Gewichtsmittelwert der Molmasse (vergleiche Fig. 13 und Fig. 14) sehen.

Mit den obigen Erklärungen kann der Mooney-Sprung auf die gesteigerte Flexibilität der hydrierten Polymerkette zurückgeführt werden.

Die Verringerung des Verhältnisses der Mooney-Viskositäten bei höheren Mooney-Viskositäten (siehe Fig. 12) kann nicht durch eine gesteigerte Flexibilität der Polymerkette erklärt werden. Die gesteigerte Flexibilität würde ein konstantes Viskositätsverhältnis von HNBR und NBR für lineare Polymerketten und ein gesteigertes Viskositätsverhältnis für verzweigte oder teilverzweigte Polymere ergeben,

Da die Bedingungen der Mooney-Messungen nicht wirklich gut definiert sind, wurde das Verhältnis der Scherviskositäten zusätzlich aus den dynamisch-mechanischen Messungen mithilfe der Gleichung

$$\eta = \lim_{\omega \to 0} \frac{|G^\star(\omega)|}{\omega}$$

berechnet und als eine Funktion der Viskosität des NBR aufgetragen (siehe Fig. 15, die Viskositäten von NBR und HNBR zeigt). Das Ergebnis ist vollkommen anders als das Ergebnis der Mooney-Messungen, Ein Anstieg der Viskosität des NBR erhöht das Verhältnis zwischen der Viskosität des HNBR und des NBR, Bei den höchsten Viskositäten des NBR erreicht das Verhältnis ein konstantes Plateau.

Die Erklärung der gegensätzlichen Ergebnisse basiert wieder auf dem Einfluss der Kettenstruktur und ihrer Flexibilität auf die Viskosität.

Für verhakte lineare Polymere ergibt eine Steigerung der Scherrate eine Verringerung der Volumenviskosität. Ein verzweigte Polymer mit einer vergleichbaren Molmasse ist durch eine höhere Viskosität bei kleinen Scherraten und durch eine stärkere Verringerung der Viskosität bei gesteigerten Scherraten gekennzeichnet.

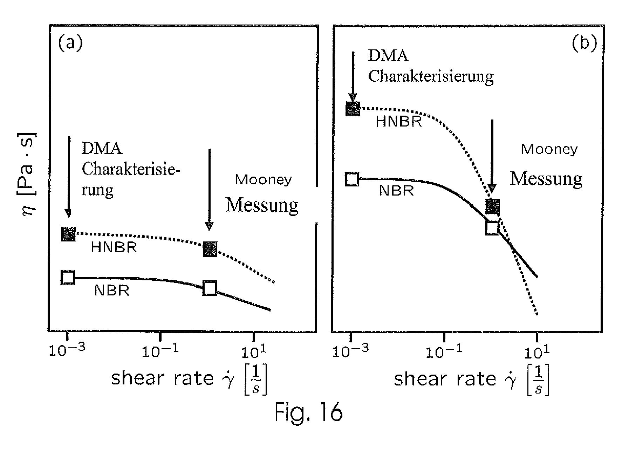

Ein schematisches Diagramm dieses Verhaltens wird in Fig. 16 (Einfluss der Scherrate auf die Viskosität für Polymere mit linearer (a) und langkettig verzweigter Kettenarchitektur) für Polymere mit verschiedenen Kettenlängen und Architektur gezeigt, Fig. 16a zeigt das Verhalten eines linearen NBR (durchgezogene Kurve) an, Die Hydrierung des NBR erhöht die Flexibilität und mithin die Viskosität (siehe gepunktete Kurve). Das Verhältnis zwischen den Viskositäten des HNBR und des NBR bei geringen und höheren Scherraten ist beinah konstant, da der Einfluss der Scherrate auf die Viskosität vergleichsweise gering für lineare Polymerketten ist.

Dies ändert sich drastisch, wenn ein NBR mit einer Fraktion von langkettlg verzweigten Ketten betrachtet wird (siehe Fig. 16b). Die Fraktion der langkettig verzweigten Ketten ergibt eine höhere Volumenviskosität bei geringen Scherraten (vergleiche durchgezogene Kurven in den Figuren 16a und 16b). Der stärkere Einfluss der Hydrierung auf die Viskosität der verzweigten Strukturen ergibt eine höhere Viskosität des HNBR bei geringen Scherraten (siehe gepunktete Kurve in Fig. 16b). Das Verhältnis zwischen den Viskositäten von HNBR und NBR bei kleinen Amplituden ist daher signifikant höher als das Viskositätsverhältnis linearer Ketten.

Da der Einfluss der Scherrate auf die Viskosität für verzweigte Ketten deutlicher ist, ist die Viskosität einer verzweigten Kette kleiner als die Viskosität einer linearen Kette in der Grenze von hohen Scherraten, Eine verzweigte Struktur im Polymer ergibt daher ein geringeres Verhältnis zwischen den Viskositäten von HNBR und NBR bei höheren Scherraten (vergleiche Viskositäten in den Figuren 16a und 16b bei einer Scherrate von

$$\dot{\gamma} \approx 10^{-3} \left[\tfrac{1}{s}\right]$$

), als ein lineares Polymer mit vergleichbarer Molmasse.

Die Ergebnisse der NR- und NBR/HNBR-Messungen zeigen, dass die DMA (also das nach der vorliegenden Erfindung durchgeführte Verfahren) ein neues Verfahren für die Charakterisierung der Molmasse und ihrer Verteilung zur Verfügung stellt, Der Vorteil der DMA gegenüber der GPC wird offensichtlich, wenn Polymerketten mit höherer Molmasse oder Fraktionen von langkettig verzweigten Strukturen vorliegen,

Da die DMA nicht kalibriert durchgeführt worden ist, können die Ergebnisse nicht für eine quantitative Bestimmung der Molmasse verwendet werden. Eine grobe Abschätzung der Molmassen wurde jedoch durch einen Vergleich der DMA- und GPC-Ergebnisse erreicht. Dies wird in den Figuren 9, 10 und 11 gezeigt, in denen Spitzenwerte der Molmassen aus GPC- und DMA-Messungen als Vergleichsgrundlage verwendet wurden,

Die Kalibrierung der GPC wird gewöhnlich mit einer Serie von Polystyrol- (oder Polyisopren-) Proben mit variierender Molmasse und enger Molmassenverteilung durchgeführt. Allgemein wird die Molmasse eines unbekannten Polymers auf der Basis der Polystyrol-Kalibrierung berechnet. Wenn die Polymer-Lösungsmittel-Wechselwirkung oder die Kettenflexibilität eines charakterisierten Polymers verschieden von Polystyrol ist (oder Polyisopren), tritt eine systematische Abweichung der Molmasse auf. Die GPC-Messungen ergeben dann nur ein qualitatives Ergebnis für die Molmasse und ihre Verteilung.

Eine Kalibrierung des DMA kann bei Bedarf ähnlich dem GPC-Verfahren durchgeführt werden. Der Vergleich der Kalibrierung mit verschiedenen Polymeren (z.B. Polystyrol und Polyisopren) würde nicht nur eine quantitative Charakterisierung ihrer Molmassen ermöglichen, sondern zusätzlich eine direkte Information über ihre Kettenflexibilität liefern.

**Patentansprüche**

1.  Verfahren zur Ermittlung einer Molekulargewichtsverteilung in einem Polymer, in dem das komplexe Schermodul in Abhängigkeit von der Frequenz gemessen wird, an das gemessene komplexe Schermodul oder an die aus dem gemessenen komplexen Schermodul ermittelte Masterkurve eine analytische Funktion angepasst wird und hieraus die Molekulargewichtsverteilung berechnet wird.

2.  Verfahren nach Anspruch 1, bei dem eine Summe von Cole-Cole Funktionen als analytische Funktion gewählt wird, die an das gemessene komplexe Schermodul oder an die aus dem gemessenen komplexen Schermodul ermittelte Masterkurve angepasst wird.

3.  Verfahren nach dem vorhergehenden Anspruch, bei dem als Breitenparameter der Cole-Cole Funktionen b=0,5 gewählt wird.

4.  Verfahren nach einem der vorhergehenden Ansprüche, bei dem eine Summe von wenigstens drei Cole-Cole Funktionen gemäß

$$F_{CCV}^{\star}(f) = \sum_{\nu=0}^{4} F_{0\nu} \frac{\sqrt{\imath \dfrac{f}{f_{0\nu}}}}{1 + \sqrt{\imath \dfrac{f}{f_{0\nu}}}} \quad {}^{n}$$

analytisch an das numerisch vorliegende, frequenzabhängig gemessene komplexe Schermodul oder an eine numerisch vorliegendende Masterkurve angepasst wird und hiervon ausgehend das Relaxationszeitspektrum berechnet wird, wobei die Parameter $F_{0\nu}$, und $f_{0\nu}$ so gewählt werden, dass die Summe der Cole-Cole-Funktionen ergibt: Der Realteil hat in der doppelt logarithmischen Darstellung im Grenzfall kleiner Frequenzen die Steigung 2 und der Imaginärteil hat im Grenzfall kleiner Frequenzen die Steigung 1,

5.  Verfahren nach einem der vorhergehenden Ansprüche, bei dem aus der analytischen Funktion das Relaxationszeitspektrum ermittelt und mit dem Relaxotionszeitspektrum und der verallgemeinerten Mischungsregel die Molekulargewichtsverteilung ermittelt wird.

6. Verfahren nach einem der vorhergehenden Ansprüche, bei dem das komplexe Schermodul bei verschiedenen Temperaturen gemessen wird und aus den gemessenen Werten eine Masterkurve gebildet wird.

7. Verfahren nach einem der vorhergehenden Ansprüche, bei dem die verallgemeinerte Mischungsregel

$$w(m) = \frac{1}{\beta} \left( \frac{\alpha}{G_0} \right)^{\frac{1}{\beta}} h(m) \left[ \int_{\ln m}^{\infty} h(m') \, d\ln m' \right]^{\frac{1}{\beta}-1}$$

zur Ermittlung der Molekulargewichtsverteilung verwendet wird.

8. Verfahren nach einem der vorhergehenden Ansprüche, bei dem ein Relaxationszeitspektrum um die Segmentrelaxationen des Glasprozesses bereinigt wird.

$$M = (M_0 - M_\infty) \cdot e^{-\frac{n}{n_0}} + M_\infty$$

$$M_0 = 78.3(\pm 3) \text{ MU}$$
$$M_\infty = 4.2(\pm 1) \text{ MU}$$
$$n_0 = 24.1(\pm 2)$$

Fig. 1

Fig. 2

Fig. 3

Fig. 4

Fig. 5

Fig. 6

Fig. 7

$$\tau_i = \frac{1}{2\pi f_i}$$

Fig. 8

[beliebige Einheiten]

Fig. 9

molare Masse von DMA Messungen [beliebige Einheiten]

Fig. 10

molare Masse von DMA Messungen [beliebige Einheiten]

Fig. 11

Fig. 12

Fig. 13

Fig. 14

Fig. 15

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **W. THIMM et al.** An analytical relation between relaxation time spectrum and molecular weight distribution. *J. Rheol,* 1999, vol. 43 (6), 1663-1672
- **COLE R. ; COLE H.** *J. Chem Phys,* 1941, vol. 9, 341
- **FREIBURG.** Material Research Center. NLREG for nonlinear regularization. *Version Rheology 2.0,* 2001
- **THIMM W ; FRIEDRICH C ; HONERKAMP J.** *J. Rheol.,* 1999, vol. 6, 43
- **ROUSE, P. E.** *J. Chem, Phys.,* 1953, vol. 21, 1272